# EUROPEAN PATENT SPECIFICATION

(11) **EP 2 481 412 B1**
(45) Date of publication and mention of the grant of the patent: **27.09.2017**
(21) Application number: 12165557.5
(22) Date of filing: 25.05.2005
(51) Int. Cl.: A61K 31/4174, A61K 31/4164, A61P 17/00, A61P 17/10

(54) **Compounds, formulations, and methods for treating or preventing inflammatory skin disorders**
Verbindungen, Formulierungen und Verfahren zur Behandlung oder Vorbeugung entzündlicher Hautstörungen
Composés, formulations et procédés pour traiter ou prévenir les troubles inflammatoires de la peau

(30) Priority: 25.05.2004 US 853585; 25.05.2004 US 574142 P
(43) Date of publication of application: 01.08.2012
(62) Divisional of application: 05754754.9
(73) Proprietor: Galderma Pharma S.A., 1000 Lausanne 30 Grey (CH)
(72) Inventor: DeJovin, Jack A., New Brunswick, NJ New Jersey 08901 (US); Rossi, Thomas, M., Stockton, NJ New Jersey 08559 (US)
(74) Representative: V.O.

(56) References cited:
- WO-A-2004/105703
- WO-A-2005/002580
- WO-A-2005/010025
- WO-A1-99/26942
- US-A- 4 256 763
- US-A- 4 285 967
- US-A- 5 916 574
- GUARRERA M ET AL: "FLUSHING IN ROSACEA: A POSSIBLE MECHANISM", ARCHIVES OF DERMATOLOGICAL RESEARCH, SPRINGER, INTERNATIONAL, BERLIN, DE, vol. 272, no. 3/04, 1 January 1982 (1982-01-01), pages 311-316, XP008012346, ISSN: 0340-3696
- REBORA A: "THE MANAGEMENT OF ROSACEA", AMERICAN JOURNAL OF CLINICAL DERMATOLOGY, ADIS, US, vol. 3, 1 January 2002 (2002-01-01), pages 489-496, XP009034164, ISSN: 1175-0561
- BURKE J ET AL: "Preclinical evaluation of brimonidine", SURVEY OF OPHTHALMOLOGY, SURVEY OF OPHTHALMOLOGY INC, XX, vol. 41, 1 November 1996 (1996-11-01), pages S9-S18, XP023437195, ISSN: 0039-6257 [retrieved on 1996-11-01]
- NIELSEN H ET AL: "POSTJUNCTIONAL ALPHA-2-ADRENOCEPTORS MEDIATE VASOCONSTRICTION IN HUMAN SUBCUTANEOUS RESISTANCE VESSELS", BRITISH JOURNAL OF PHARMACOLOGY, vol. 97, no. 3, 1989, pages 829-834, XP009114426, ISSN: 0007-1188
- LINDGREN B R ET AL: "EFFECTS OF SOME ANTIHYPERTENSIVE DRUGS ON CUTANEOUS BLOOD FLOW AND INFLAMMATORY SKIN RESPONSES FOLLOWING ALLERGEN CHALLENGE IN GUINEA-PIGS", PHARMACOLOGY AND TOXICOLOGY, vol. 60, no. 5, 1987, pages 364-367, XP002546987, ISSN: 0901-9928
- ANDERSON C D ET AL: "EFFECTS OF CLONIDINE ON THE DERMAL INFLAMMATORY CELL RESPONSE OF EXPERIMENTAL TOXIC AND ALLERGIC CONTACT REACTIONS AND INTRADERMAL HYPERSENSITIVITY", INTERNATIONAL ARCHIVES OF ALLERGY AND APPLIED IMMUNOLOGY, BASEL, CH, vol. 83, no. 4, 1 January 1987 (1987-01-01), pages 371-376, XP009123072, ISSN: 0020-5915
- DATABASE BIOSIS [Online] BIOSCIENCES INFORMATION SERVICE, PHILADELPHIA, PA, US; 1991, NABE T ET AL: "ANTI-INFLAMMATORY ACTION OF GUANABENZ", XP002677210, Database accession no. PREV199192056514 & OYO YAKURI, vol. 41, no. 4, 1991, pages 327-340, ISSN: 0300-8533

## Description

### Field Of The Invention

The present invention is directed to compositions for use in the topical treatment of an inflammatory skin disorder as defined in appended claim 1. The compounds and methods described herein are particularly useful for treating inflammatory skin disorders and the symptoms associated therewith.

### Background Of The Invention

Many people are affected by inflammatory skin disorders that result in unsightly and painful rashes, acne, persistent red veins, and acne-like skin eruptions, such as macules, nodules, and pustules that may ooze or crust. Inflammatory skin disorders often result in intense psychosocial distress. Rosacea is a common inflammatory skin disorder affecting over 10 million people in the United States. Rosacea generally involves the cheeks, nose, chin, and forehead and the typical age of onset is 30 to 60 years. See e.g., Zuber T.J., Rosacea: Beyond First Blush 32 HOSP. PRACT. 188-189 (1997); THE MERCK MANUAL 813-814 (Keryn A.G. Lane et al. eds. 17th ed. 2001). Many people with early-stage rosacea incorrectly assume that they suffer from adult acne, sun or windburn, or the normal effects of aging.

Rosacea develops gradually starting as frequent blushing and frequent irritation of the facial skin.

More advanced rosacea is characterized by a vascular stage where patients display increasingly severe erythema (abnormal redness of the skin) and telangiectasia (visible red lines due to abnormal dilatation of capillary vessels and arterioles). Pimple-like eruptions, which may be solid (called papules or nodules) or puss filled (known as pustules) may develop. Such eruptions often look like acne, but whiteheads or blackheads (common symptoms of acne) are not normally present. Later-stage rosacea is characterized by rhinophyma (enlargement of the nose). If left untreated, rosacea can progress to irreversible disfigurement. Rosacea symptoms are often aggravated by sun exposure, changes or extremes in temperature, wind, and consumption of certain foods, such as spicy foods, caffeine, and alcohol.

The exact pathogenesis of rosacea is unknown, but the pathologic process is well described. For example, erythema associated with rosacea is caused by dilation of the superficial vasculature of the face. Zuber T.J., Rosacea: Beyond First Blush 32 HOSP. PRACT. 188-189 (1997).

There is no known cure for many inflammatory skin disorders like rosacea. Current treatments, which are directed to control of redness, inflammation, and skin eruptions, are of limited effectiveness in many patients and, generally, can be used only for a limited duration. Standard treatments include avoidance of triggers such as sun exposure, wind exposure, alcohol consumption, spicy foods, and irritating facial cleansers, lotions, and cosmetics. Antibiotics are the traditional first line of therapy. Long-term treatment (5 to 8 weeks or more) with oral antibiotics such as tetracycline, minocycline, doxycycline or clarithromycin may control skin eruptions. Alternative oral treatments include vitamin A medications, such as isoretinoin and antifungal medications. Unfortunately, such oral medications often cause side effects and many people have limited tolerance. Topical treatments, such at topically applied antibiotics and antifungals (such as metronidazole) or steroids, are available but also have limited effectiveness and cannot treat all symptoms. For example, isoretinoin has serious teratogenic side-effects and female patients of child bearing age must use effective birth control or avoid the therapy. Topical treatments include topically applied metronidazole, topically applied steroids, topically applied azelaic acid, topically applied rentinoic acid or retinaldehyde, and topical vitamin C preparations are available but have limited effectiveness and cannot treat all symptoms. Surgery, such as the laser elimination of blood vessels, is typically a last resort, but may be prescribed if other treatments are ineffective. In patients with nose hyperplasia, surgical reduction may improve the patient's cosmetic appearance, but does not treat the disease itself. Mixed light pulse (photoderm) therapy has proved somewhat effective for symptoms associated with certain inflammatory skin orders like rosacea in some patients. Thus, there remains a need for topical formulations for treatment of inflammatory skin disorders like rosacea and its symptoms.

Agonists of the α₂ adrenoceptors have been used therapeutically for a number of conditions including hypertension, congestive heart failure, angina pectoris, spasticity, glaucoma, diarrhea, and for the suppression of opiate withdrawal symptoms (J.P. Heible and R.R. Ruffolo Therapeutic Applications of Agents Interacting with α-Adrenoceptors, p. 180-206 in Progress in Basic and Clinical Pharmacology Vol. 8, P. Lomax and E.S. Vesell Ed., Karger, 1991).

Adrenoreceptor agonists such as clonidine have been primarily used orally, though a patch formulation is known. The goal of existing formulations is to deliver a systemic internal dose of the compound to the patient. The α₂ agonists are known to mediate vasoconstriction both in the core and periphery of a patient. In particular α₂ adrenoceptor agonists are known to cause vasoconstriction of peripheral arterioles, in response to stimulation due to cold or stress.

A number of patents describe the use of brimonidine for treating ophthalmic conditions and eye diseases. In Canadian patent No. CA2326690, there is described the use of topical ophthalmic preparations for use only in the eyes, to treat eye diseases. The Canadian patent discusses the problems with ophthalmic preparations taken topically (in the eye), orally or parenterally, and their systemic effects, including some serious, that limit their use. These systemic effects include, cardiopulmonary effects of β-blockers like timolol; dryness of mouth, flush, fever, tachy cardia, urinary retention, convulsion and irritability with atropine; hypertension with phenylephine; increased salivation, nausea, vomiting, diarrhea, stomach cramps, bronchial secretions, brionchial constriction, asthma, bradycardia, paresthesia with miotics; hypotension with clonidine; and dry mouth, fatigue and drowsiness with apraclonidine and brimonidine.

There has been no composition containing α₂ adrenoceptor agonists that can deliver a dose of the agonist to the patient, ameliorating the symptoms of rosacea or other inflammatory skin disorders, without causing systemic side effects. There has also been no topical skin composition containing α₂ adrenoceptor agonists that can deliver a dose of the agonist to the skin of the patient, ameliorating the symptoms of rosacea and/or other inflammatory skin disorders, without causing systemic side effects.

WO 2005/002580 discloses a method of preventing or reducing the severity of a stress-associated inflammatory dermatological condition in a subject by systemically administering brimonidine or a pharmaceutically acceptable salt, ester, amide, stereoisomer or racemic mixture thereof. The European patent application (publication No. EP 1638569) derived from the foregoing international patent application is comprised in the state of the art within the meaning of Article 54(3) EPC.

WO 2004/105703 discloses a method of treating or preventing rosacea and the symptoms associated therewith by topically administering to the skin of a patient a composition comprising a therapeutically effective amount of at least one of an α2 adrenergic receptor agonist and a pharmaceutically acceptable salt thereof; and a pharmaceutically acceptable carrier. The European patent application (publication No. EP 1631293) derived from the foregoing international patent application is comprised in the state of the art within the meaning of Article 54(3) EPC.

U.S. Patent No. 4,256,763 discloses a combination comprising phenolphthalein and phenylephrine for the treatment of acne, whereby the combination is administered orally.

U.S. Patent No. 5,916,574 discloses a natural poison skin treatment composition which includes (i) a natural poison skin treatment medication selected from benzoyl peroxide and salicyclic acid; (ii) a vasoconstrictor selected from, amongst others, catecholamines, norepinephrine, epinephrine, isoproterenol, dopamine, ephedrine, phenylisopropoylamines, phenylephrine, amphetamine, metraminol, methoxamine, lysergic acid, and lysergic acid diethyamine; and (iii) an inert carrier. The vasoconstrictor is stated to be used to remove the redness associated with the natural poison skin condition, such as poison oak, poison ivy and poison sumac.

U.S. Patent No. 4,285,967 discloses cosmetic compositions comprising phenylephrine hydrochloride which are stated to be effective for reducing the redness of facial blemishes by constricting the small broken capillaries on the skin making redness less obvious.

Lindgren B. R. et al.: "Effects of some antihypertensive drugs on cutaneous blood flow and inflammatory skin responses following allergen challenge in guinea-pigs", Pharmacology and Toxicology, Vol. 60, No. 5, 1987, pp. 364-367 describes the result of a study undertaken to investigate the effects of the antihypertensive drugs, clonidine, prazosin and MK 422, on cutaneous blood flow and allergen-induced inflammatory skin responses. The tests involved administering the drugs by intraperitonial injections twice a day.

### Summary Of The Invention

The present invention provides compositions and topical skin formulations for use in the topical treatment of the inflammatory skin disorder of dermatitis and symptoms associated therewith in accordance with appended claim 1. The compounds underlying the invention are particularly effective for treatment of dermatitis, such as contact dermatitis, atopic dermatitis, seborrheic dermatitis, nummular dermatitis, generalized exfoliative dermatitis, statis dermatitis, and lichen simplex chronicus. Compounds underlying the invention are α₂ adrenoceptor agonists that act on the peripheral vasculature to cause vasoconstriction and thereby ameliorate the symptoms of inflammatory skin disorders. The compounds are delivered in a topical skin composition that insures that the compounds are effective in the skin of a patient but do not penetrate the skin in sufficient amounts to induce serious systemic side effects.

Compounds underlying the invention are selected from naphazoline, xylometazoline and their respective pharmaceutically acceptable salts.

To treat the inflammatory skin disorder of dermatitis, the compositions underlying the invention are to be topically applied. Formulations for topical delivery of the compounds underlying the invention are well-known in the art, such as aqueous or non-aqueous solutions or suspensions, creams, lotions, gels, or ointments.

These and other features, aspects, and advantages of the invention will become better understood with reference to the following detailed description, examples, and appended claims.

### Detailed Description

### 1.1 COMPOUNDS UNDERLYING THE INVENTION

The α₂ adrenergic receptor agonist and/or a pharmaceutically acceptable salt thereof underlying the compositions for use according to the present invention are listed in Table 1 below.

**Table 1: Compounds Underpins The Invention**

| Compound underlying the Invention | Name |
|---|---|
| 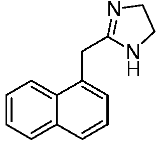 | Naphazoline |
| 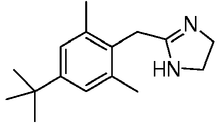 | Xylometazoline |

The compounds underlying the invention are well known in the art to be α₂ adrenergic receptor agonists. As such the compounds have powerful vasoconstricting effects when introduced into the body of mammals, particularly humans.

### 1.2 SYNTHESIS OF COMPOUNDS UNDERLYING THE INVENTION

The compounds underlying the invention can be prepared in accordance with well-known synthetic procedures, for example, using the general synthetic procedures outlined in U.S. Patent Nos. 3,890,319 (issued June 17, 1975) and 4,029,792 (issued June 14, 1977).

### 1.3 TOPICAL FORMULATIONS UNDERLYING THE INVENTION

The compounds described above are to be delivered to the affected area of the skin in a pharmaceutically acceptable topical carrier. As used herein, a pharmaceutically acceptable topical carrier is any pharmaceutically acceptable formulation that can be applied to the skin surface for topical, dermal, intradermal, or transdermal delivery of a pharmaceutical or medicament. The combination of a pharmaceutically acceptable topical carrier and a compound described above is termed a topical formulation underlying the invention. Topical formulations underlying the invention are prepared by mixing a compound described above with a topical carrier according to well-known methods in the art, for example, methods provided by standard reference texts such as, REMINGTON: THE SCIENCE AND PRACTICE OF PHARMACY 1577-1591, 1672-1673, 866-885(Alfonso R. Gennaro ed. 19th ed. 1995); Ghosh, T. K.; et al. TRANSDERMAL AND TOPICAL DRUG DELIVERY SYSTEMS (1997).

The topical carriers useful for topical delivery of compounds described above can be any carrier known in the art for topically administering pharmaceuticals, for example, but not limited to, pharmaceutically acceptable solvents, such as a polyalcohol or water; emulsions (either oil-in-water or water-in-oil emulsions), such as creams or lotions; micro emulsions; gels; ointments; liposomes; powders; and aqueous solutions or suspensions, such as standard ophthalmic preparations.

### 1.3.1 Emulsions, Gels, and Ointments As Topical Carriers

In a preferred embodiment, the topical carrier used to deliver a compound described above is an emulsion, gel, or ointment. Emulsions, such as creams and lotions are suitable topical formulations for use in accordance with the invention. An emulsion is a dispersed system comprising at least two immiscible phases, one phase dispersed in the other as droplets ranging in diameter from 0.1 µm to 100 µm. An emulsifying agent is typically included to improve stability. When water is the dispersed phase and an oil is the dispersion medium, the emulsion is termed a water-in-oil emulsion. When an oil is dispersed as droplets throughout the aqueous phase as droplets, the emulsion is termed an oil-in-water emulsion. Emulsions, such as creams and lotions that can be used as topical carriers and their preparation are disclosed in REMINGTON: THE SCIENCE AND PRACTICE OF PHARMACY 282-291 (Alfonso R. Gennaro ed. 19th ed. 1995).

In another embodiment, the topical carrier used to deliver a compound described above is a gel, for example, a two-phase gel or a single-phase gel. Gels are semisolid systems consisting of suspensions of small inorganic particles or large organic molecules interpenetrated by a liquid. When the gel mass comprises a network of small discrete inorganic particles, it is classified as a two-phase gel. Single-phase gels consist of organic macromolecules distributed uniformly throughout a liquid such that no apparent boundaries exist between the dispersed macromolecules and the liquid. Suitable gels for use in the invention are disclosed in REMINGTON: THE SCIENCE AND PRACTICE OF PHARMACY 1517-1518 (Alfonso R. Gennaro ed. 19th ed. 1995). Other suitable gels for use with the invention are disclosed in U.S. Patent Nos. 6,387,383 (issued May 14, 2002); 6,517,847 (issued Feb. 11, 2003); and 6,468,989 (issued Oct. 22, 2002).

Polymer thickeners (gelling agents) that may be used include those known to one skilled in the art, such as hydrophilic and hydroalcoholic gelling agents frequently used in the cosmetic and pharmaceutical industries. Preferably, the hydrophilic or hydroalcoholic gelling agent comprises "CARBOPOL®" (B.F. Goodrich, Cleveland, Ohio), "HYPAN®" (Kingston Technologies, Dayton, N.J.), "NATROSOL®" (Aqualon, Wilmington, Del.), "KLUCEL®" (Aqualon, Wilmington, Del.), or "STABILEZE®" (ISP Technologies, Wayne, N.J.). Preferably the gelling agent comprises between about 0.2% to about 4% by weight of the composition. More particularly, the preferred compositional weight percent range for "CARBOPOL®" is between about 0.5% to about 2%, while the preferred weight percent range for "NATROLSOL®" and "KLUCEL®" is between about 0.5% to about 4%. The preferred compositional weight percent range for both "HYPAN®" and "STABILEZE®" is between 0.5% to about 4%.

"CARBOPOL®" is one of numerous cross-linked acrylic acid polymers that are given the general adopted name carbomer. These polymers dissolve in water and form a clear or slightly hazy gel upon neutralization with a caustic material such as sodium hydroxide, potassium hydroxide, triethanolamine, or other amine bases. "KLUCEL®" is a cellulose polymer that is dispersed in water and forms a uniform gel upon complete hydration. Other preferred gelling polymers include hydroxyethylcellulose, cellulose gum, MVE/MA decadiene crosspolymer, PVM/MA copolymer, or a combination thereof.

In another preferred embodiment, the topical carrier used to deliver a compound described above is an ointment. Ointments are oleaginous semisolids that contain little if any water. Preferably, the ointment is hydrocarbon based, such as a wax, petrolatum, or gelled mineral oil. Suitable ointments for use in the invention are well known in the art and are disclosed in REMINGTON: THE SCIENCE AND PRACTICE OF PHARMACY 1585-1591 (Alfonso R. Gennaro ed. 19th ed. 1995).

### 1.3.2 Aqueous Topical Formulations Underling The Invention

In another embodiment, the topical carrier used in the topical formulations underlying the invention is an aqueous solution or suspension, preferably, an aqueous solution. Well-known ophthalmic solutions and suspensions are suitable topical carriers for use in the invention. Suitable aqueous topical formulations for use in the invention are disclosed in REMINGTON: THE SCIENCE AND PRACTICE OF PHARMACY 1563-1576 (Alfonso R. Gennaro ed. 19th ed. 1995). Other suitable aqueous topical carrier systems are disclosed in U.S. Patent Nos. 5,424,078 (issued Jun. 13, 1995); 5,736,165 (issued Apr. 7, 1998); 6,194,415 (issued Feb. 27, 2001); 6,248,741 (issued Jun. 19, 2001); 6,465,464 (issued Oct. 15, 2002).

The pH of the aqueous topical formulations underlying the invention are preferably within the range of from about 6 to about 8, more preferably, of from about 6.3 to about 6.5. To stabilize the pH, preferably, an effective amount of a buffer is included. In one embodiment, the buffering agent is present in the aqueous topical formulation in an amount of from about 0.05 to about 1 weight percent of the formulation. Acids or bases can be used to adjust the pH as needed. Suitable buffering agents are listed below in Section 1.3.3.

Tonicity-adjusting agents can be included in the aqueous topical formulations underlying the invention. Examples of suitable tonicity-adjusting agents include, but are not limited to, sodium chloride, potassium chloride, mannitol, dextrose, glycerin, and propylene glycol. The amount of the tonicity agent can vary widely depending on the formulation's desired properties. In one embodiment, the tonicity-adjusting agent is present in the aqueous topical formulation in an amount of from about 0.5 to about 0.9 weight percent of the formulation.

Preferably, the aqueous topical formulations of the invention have a viscosity in the range of from 0.015 to 0.025 Pa.s (about 15 cps to about 25 cps). The viscosity of aqueous solutions of the invention can be adjusted by adding viscosity adjusting agents, for example, but not limited to, polyvinyl alcohol, povidone, hydroxypropyl methyl cellulose, poloxamers, carboxymethyl cellulose, or hydroxyethyl cellulose.

In a preferred embodiment, the aqueous topical formulation underlying the invention is isotonic saline comprising a preservative, such as benzalkonium chloride or chlorine dioxide, a viscosity-adjusting agent, such as polyvinyl alcohol, and a buffer system such as sodium citrate and citric acid.

### 1.3.3 Excipients

The topical formulations underlying the invention can comprise pharmaceutically acceptable excipients such as those listed in REMINGTON: THE SCIENCE AND PRACTICE OF PHARMACY 866-885(Alfonso R. Gennaro ed. 19th ed. 1995; Ghosh, T. K.; et al. TRANSDERMAL AND TOPICAL DRUG DELIVERY SYSTEMS (1997), including, but not limited to, protectives, adsorbents, demulcents, emollients, preservatives, antioxidants, moisturizers, buffering agents, solubilizing agents, skin-penetration agents, and surfactants.

Suitable protectives and adsorbents include, but are not limited to, dusting powders, zinc sterate, collodion, dimethicone, silicones, zinc carbonate, aloe vera gel and other aloe products, vitamin E oil, allatoin, glycerin, petrolatum, and zinc oxide.

Suitable demulcents include, but are not limited to, benzoin, hydroxypropyl cellulose, hydroxypropyl methylcellulose, and polyvinyl alcohol.

Suitable emollients include, but are not limited to, animal and vegetable fats and oils, myristyl alcohol, alum, and aluminum acetate.

Suitable preservatives include, but are not limited to, quaternary ammonium compounds, such as benzalkonium chloride, benzethonium chloride, cetrimide, dequalinium chloride, and cetylpyridinium chloride; mercurial agents, such as phenylmercuric nitrate, phenylmercuric acetate, and thimerosal; alcoholic agents, for example, chlorobutanol, phenylethyl alcohol, and benzyl alcohol; antibacterial esters, for example, esters of parahydroxybenzoic acid; and other anti-microbial agents such as chlorhexidine, chlorocresol, benzoic acid and polymyxin.

Chlorine dioxide (ClO₂), preferably, stabilized chlorine dioxide, is a preferred preservative for use with topical formulations underlying the invention. The term "stabilized chlorine dioxide" is well known in the industry and by those skilled in the art. Stabilized chlorine dioxide includes one or more chlorine dioxide precursors such as one or more chlorine dioxide-containing complexes and/or one or more chlorite-containing components and/or one or more other entities capable of decomposing or being decomposed in an aqueous medium to form chlorine dioxide. U.S. Patent No. 5,424,078 (issued Jun. 13, 1995) discloses a form of stabilized chlorine dioxide and a method for producing same, which can be used as a preservative for aqueous ophthalmic solutions and is useful in topical formulations of the invention. The manufacture or production of certain stabilized chlorine dioxide products is described in U.S. Pat. No. 3,278,447. A commercially available stabilized chlorine dioxide which can be utilized in the practice of the present invention is the proprietary stabilized chlorine dioxide of BioCide International, Inc. of Norman, OK, sold under the trademark Purogene™ or Purite™. Other suitable stabilized chlorine dioxide products include that sold under the trademark DuraKlor by Rio Linda Chemical Company, Inc., and that sold under the trademark Antheium Dioxide by International Dioxide, Inc.

Suitable antioxidants include, but are not limited to, ascorbic acid and its esters, sodium bisulfite, butylated hydroxytoluene, butylated hydroxyanisole, tocopherols, and chelating agents like EDTA and citric acid.

Suitable moisturizers include, but are not limited to, glycerin, sorbitol, polyethylene glycols, urea, and propylene glycol.

Suitable buffering agents for use in the compositions underlying the invention include, but are not limited to, acetate buffers, citrate buffers, phosphate buffers, lactic acid buffers, and borate buffers.

Suitable solubilizing agents include, but are not limited to, quaternary ammonium chlorides, cyclodextrins, benzyl benzoate, lecithin, and polysorbates.

Suitable skin-penetration agents include, but are not limited to, ethyl alcohol, isopropyl alcohol, octylphenylpolyethylene glycol, oleic acid, polyethylene glycol 400, propylene glycol, N-decylmethylsulfoxide, fatty acid esters (e.g., isopropyl myristate, methyl laurate, glycerol monooleate, and propylene glycol monooleate); and N-methyl pyrrolidone.

### 1.3.4 Pharmaceutical Additives

The topical formulations underlying the invention can include pharmaceuticals or their pharmaceutically acceptable salts, for example, but not limited to, topical corticosteroids and other anti-inflammatory agents, such as betamethasone, diflorasone, amcinonide, fluocinolone, mometasone, hydrocortisone, prednisone, and triamcinolone; local anesthetics and analgesics, such as camphor, menthol, lidocaine, and dibucaine, and pramoxine; antifungals, such as ciclopirox, chloroxylenol, triacetin, sulconazole, nystatin, undecylenic acid, tolnaftate, miconizole, clotrimazole, oxiconazole, griseofulvin, econazole, ketoconozole, and amphotericin B; antibiotics and anti-infectives, such as mupirocin, erythromycin, clindamycin, gentamicin, polymyxin, bacitracin, and silver sulfadiazine; and antiseptics, such as iodine, povidine-iodine, benzalkonium chloride, benzoic acid, chlorhexidine, nitrofurazine, benzoyl peroxide, hydrogen peroxide, hexachlorophene, phenol, resorcinol, and cetylpyridinium chloride.

### 1.4 DOSAGE

Dosages and dosing frequency will be determined by a trained medical professional depending on the activity of the compound underlying the composition for use according to the invention, the characteristics of the particular topical formulation, and the identity and severity of the dermatologic disorder treated.

In general, a compound described above is present in a formulation for use according to the invention in an amount of from about 0.01 percent to about 5 percent of the total weight of the formulation, preferably, of from about 0.05 percent to about 1 percent, more preferably, of from about 0.1 percent to about 0.2 percent of the total weight of the formulation.

To treat or prevent the inflammatory skin disorder specified in the appended claims, the topical formulations underlying the invention are topically applied directly to the affected area in any conventional manner well known in the art. For example, by dropper or applicator stick, as a mist via an aerosol applicator, via an intradermal or transdermal patch, or by simply spreading a formulation of the invention onto the affected area with fingers. Generally the amount of the topical formulation applied to the affected skin area ranges from about 0.1 g/cm² of skin surface area to about 5 g/cm², preferably, 0.2 g/cm² to about 0.5 g/cm² of skin surface area. Typically, one to four applications per day are recommended during the term of treatment.

### 1.5 USE OF TOPICAL FORMULATIONS UNDERLYING THE INVENTION IN COMBINATION WITH OTHER SKIN-DISORDER TREATMENTS

The formulations underlying the invention can be used in combination with other treatments and medications to provide more effective treatment of the inflammatory skin disorder and symptoms associated therewith. In a preferred embodiment, the topical formulations underlying the invention can be used in combination with treatment regimens and medications well known for treatment of dermatologic disorders, such as those disclosed in THE MERCK MANUAL 811-830 (Keryn A.G. Lane et al. eds. 17th ed. 2001).

Using a formulation underlying the invention in combination with another medicament or treatment means administering a formulation underlying the invention and the other medicament or treatment to a subject in a sequence and within a time interval such that they can act together to treat the inflammatory skin disorder and symptoms associated therewith. For example, the compounds underlying the compositions for use according to the invention can be administered at the same time as the other medicament in the same or separate formulations or at different times.

Any suitable route of administration can be employed to deliver the additional treatment or medication including, but not limited to, oral, intraoral, rectal, parenteral, topical, epicutaneous, transdermal, subcutaneous, intramuscular, intranasal, sublingual, buccal, intradural, intraocular, intrarespiratory, or nasal inhalation. Thus, the formulations underlying the invention can be administered together or at separate times with other medications or treatments.

In one embodiment, the topical formulations underlying the invention are used in combination with systemic administration of antibiotics or retinoids including, but not limited to, orally dosed antibiotics, such as tetracycline, minocin, minocycline, erythromycin, and doxycycline, and orally dosed retinoids such as isotretinoins (e.g., Accutane or Roaccutance).

In another embodiment, the topical formulations underlying the invention are used in combination with other topical treatments including, but not limited to, topical formulations consisting of metronidizole, hydrogen peroxide, benzoyl peroxide, lipoic acid, and azelaic acid, and sulfur preparations; topically dosed antibiotics, such as metronidazole, clindamycin, and erythromycin; topical retinoids such as tretinoin, adapalene, tazarotene; or topical steroids.

In another embodiment, the topical formulations underlying the invention are used in combination with mixed light pulse therapy (photoderm), pulsed dye laser treatment, or electrosurgery.

### 1.6 ARTICLE OF MANUFACTURE

Described herein is an article of manufacture that comprises a topical formulation underlying the invention in a suitable container with labeling and instructions for use. The container can be a dropper or tube with a suitable small orifice size, such as an extended tip tube made of any pharmaceutically suitable material.

The topical formulations can be filled and packaged into a plastic squeeze bottle or tube. Suitable container-closure systems for packaging topical formulations underlying the invention are commercially available for example, from Wheaton Plastic Products, 1101 Wheaton Avenue, Millville, NJ 08332.

Preferably, instructions are packaged with the formulations underlying the invention, for example, a pamphlet or package label. The labeling instructions explain how to administer topical formulations underlying the invention, in an amount and for a period of time sufficient to treat the inflammatory skin disorder and symptoms associated therewith. The labeling instructions are an important aspect of the invention in that before a composition can be approved for any particular use, it must, for example, be approved for marketing by the United States Food and Drug Administration. Part of that process includes providing a label that will accompany the pharmaceutical composition that is ultimately sold. Preferably, the label includes the dosage and administration instructions, the topical formulation's composition, the clinical pharmacology, drug resistance, pharmacokinetics, absorption, bioavailability, and contraindications.

### 1.7 EXAMPLES

The following examples are provided for illustrative purposes only and are not to be construed as limiting the invention's scope in any manner.

### 1.7.1 Example 1: Synthesis of (5-Bromo-guinoxalin-6-yl)-(4,5-dihydro-1H-imidazol-2-yl)-amine (not within scope of appended claims)

To a stirred solution of 6-amino-5-bromoquinoxaline hydrobromide (10 g) in distilled water (150 ml) is added thiophosgene (3 ml). The solution is stirred for two hours at room temperature and the resultant precipitate is collected by filtration, washed with water, and dried to afford 5-bromo-6-isothiocyanato-quinoxaline.

The 5-bromo-6-isothiocyanato-quinoxaline (3.5 g) is directly dissolved in benzene (400 ml) and added dropwise to a well-stirred solution of ethylene diamine (15 g) in benzene (50 ml). During a period of about two hours, an oil separates as a lower layer. The upper benzene layer is poured off and the oil is washed with diethyl ether and then dissolved in methanol (500 ml). The methanolic solution is refluxed until hydrogen sulfide evolution ceases. The methanolic solution is concentrated *in vacuo* to a volume of approximately 100 ml upon which a yellow solid precipitates. The precipitate is collected by filtration and recrystallized from methanol to afford of (5-Bromo-quinoxalin-6-yl)-(4,5-dihydro-1H-imidazol-2-yl)-amine: m.p. 250-251 C.

### 1.7.2 Example 2 (not within scope of appended claims)

An aqueous solution topical formulation comprises (5-Bromo-quinoxalin-6-yl)-(4,5-dihydro-1H-imidazol-2-yl)-amine-L-tartrate (brimonidine tartrate) (0.15 wt. %); Purite® (0.005%) (stabilized chlorine dioxide) as a preservative; and the inactive ingredients: boric acid; calcium chloride; magnesium chloride; potassium chloride; purified water; sodium borate; sodium carboxymethylcellulose; sodium chloride; with hydrochloric acid and/or sodium hydroxide to adjust the pH to 5.6 to 6.6. The osmolality is in the range of 250-350 mOsmol/kg.

### 1.7.3 Example 3 (not within scope of appended claims)

A aqueous solution topical formulation comprises (5-Bromo-quinoxalin-6-yl)-(4,5-dihydro-1H-imidazol-2-yl)-amine-L-tartrate, (brimonidine tartrate) (0.15 wt. %); benzalkonium chloride (0.005 wt. %) as a preservative; and the inactive ingredients: boric acid; calcium chloride; magnesium chloride; potassium chloride; purified water; sodium borate; sodium carboxymethylcellulose; sodium chloride; with hydrochloric acid and/or sodium hydroxide to adjust the pH to 5.6 to 6.6. The osmolality is in the range of 250-350 mOsmol/kg.

### 1.7.4 Example 4 (not within scope of appended claims)

A possible cream topical formulation is described in the Table below.

**Possible Cream Formulation (Hydrophilic Ointment USP)**

| Ingredient | Weight Percent |
|---|---|
| Brimonidine tartrate | 0.15% |
| Stearic acid | 7% |
| Stearyl alcohol | 5% |
| Cetyl alcohol | 2% |
| Glycerin | 10% |
| Sodium lauryl sulfate | 1% |
| Propylparaben | 0.05% |
| Methylparaben | 0.25% |
| Disodium edetate | 0.055 |
| Distilled water | QS |

Melt the stearyl alcohol and the white petrolatum on a steam bath, and warm to about 75 degrees C. Add the other ingredients, previously dissolved in the water and warmed to 75 degrees C, and stir the mixture until it congeals. With stirring, allow the mixture to cool and add brimonidine tartrate as a concentrated solution.

### 1.7.5 Example 5 (not within scope of appended claims)

A possible ointment topical formulation is described in the Table below.

**Possible Ointment Formulation (Hydrophilic Ointment USP)**

| Ingredients | Weight |
|---|---|
| Brimonidine tartrate | 10g |
| Cholesterol | 30g |
| Stearyl Alcohol | 30g |
| White Wax | 80g |
| White Petrolatum | 850g |

Mix the stearyl alcohol and white wax together on a steam bath, then add the cholesterol and stir until it completely dissolves. Add the white petrolatum and mix. Remove from the bath, and stir until the mixture congeals. Continue stirring and add brimonidine tartrate as a concentrated slurry.

### 1.7.6 Example 6 (not within scope of appended claims)

A possible gel formulation is described in the table below.

**Possible Gel Formulation**

| Ingredients | Weight % |
|---|---|
| Brimonidine tartrate | 1.0% |
| Methylparaben NF | 0.15% |
| Propylparaben NF | 0.03% |
| Hydroxyethylcellulose NF | 1.25% |
| Disodium Edetate USP | 0.05% |
| Purified Water, USP | QS 100% |

### 1.7.7 Example 7 (not within scope of appended claims)

A possible gel formulation is described in the Table below.

**Possible Gel Formulation**

| Ingredients | Weight % |
|---|---|
| Brimonidine tartrate | 1.0% |
| Methylparaben | 0.20% |
| Propylparaben | 0.05% |
| Carbomer 934P NF | 1.0% |
| Sodium Hydroxide | QS pH 7 |
| Purified Water USP | QS 100% |

The ingredients are mixed together and aqueous sodium hydroxide is slowly added to the mixture until a pH of about 7 is reached and the gel is formed.

### 1.7.8 Example 8 (not within scope of appended claims)

A possible gel formulation is described in the Table below.

**Possible Gel Formulation**

| Ingredients | Weight % |
|---|---|
| Brimonidine tartrate | 1.0% |
| Methylparaben | 0.2% |
| Propylparaben | 0.05% |
| "CARBOPOL®" | 1.0% |
| Triethanolamine | QS pH 7 |
| Water | QS 100 % |

The ingredients are mixed together and stirred. Triethanolamine is added until a pH of about 7 is attained.

### 1.7.9 Example 9 (Reference Example)

### Testing Procedure For Prevention of Redness By α-Adrenergic Agonists:

A number of α-adrenergic agonists were evaluated for their ability to topically suppress erythema in human skin induced by methyl nicotinate. The erythema produced in the skin results from the vasodilatory effect on the dermal vasculature by methyl nicotinate. In this model, the minimum erythemal dose (MED) produced on the forearm by methyl nicotinate is determined for each test subject. The MED is defined as the minimal dose that results in a defined circle of erythema. The MED was determined by saturating five 19 mm Hill Top Chambers with 220 µl of 1, 2, 3, 4, and 5 mm methyl nicotinate. The Hill Top Chambers were applied to the volar forearm of each test subject, removed after 30 seconds and excess liquid lightly blotted from the skin. The MED of methyl nicotinate was selected 10 minutes after application, by determining the minimal dose that resulted in a defined circle of erythema. The α-adrenergic agonists were dissolved in alcohol and topically applied (2µl/cm²) to selected sites on the contralateral volar forearm for 30 minutes prior to challenge with methyl nicotinate. Hill Top Chambers (19 mm) were saturated with 220 µl of the dose of methyl nicotinate determined to produce a MED for each test subject. The chambers were applied to the volar forearm treated with vehicle or test compounds, removed after 30 seconds and excess liquid was lightly blotted from the skin. Ten minutes after application of methyl nicotinate the test sites were evaluated for erythema. A numerical grading scale of 0 to 3 was used: 0=none, 0.5=barely perceptible, 1.0=mild, 1.5=mild+ (mild to moderate), 2.0=moderate, 2.5=moderate+ (moderate to severe), 3.0=severe.

The test results are shown in the table below and indicate that each of the tested compounds reduced the formation of the Methyl Nicotinate induced redness (erythema) in the test subjects. With both Oxymetazoline HCl and Naphazoline HCl the redness was fully blocked for two of the three subjects pursuant to the test conditions as described above.

**The Effect of α-Adrenergic Agonists on Methyl Nicotinate-Induced Erythema**

| Pre-Treatment + Methyl Nicotinate | N | Mean Erythema Grade |
|---|---|---|
| Alcohol Vehicle Control | 3 | 3.0 |
| 0.2% Naphazoline HCl | 3 | 0.33 |
| 0.2% Oxymetazoline HCl | 3 | 1.0 |
| 0.2% Brimonidine | 3 | 0.83 |

### 1.8 DEFINITIONS

As used herein, the terms "an inflammatory skin disorder" and "an inflammatory dermatologic disorder" mean dermatitis, such as contact dermatitis, atopic dermatitis, seborrheic dermatitis, nummular dermatitis, generalized exfoliative dermatitis, statis dermatitis, and lichen simplex chronicus.

The phrase "pharmaceutically acceptable salt(s)", as used herein, means those salts of compounds underlying the invention that are safe and effective for topical use in mammals and that possess the desired biological activity. Pharmaceutically acceptable salts include salts of acidic or basic groups present in compounds of the invention. Pharmaceutically acceptable acid addition salts include, but are not limited to, hydrochloride, hydrobromide, hydroiodide, nitrate, sulfate, bisulfate, phosphate, acid phosphate, isonicotinate, acetate, lactate, salicylate, citrate, tartrate, pantothenate, bitartrate, ascorbate, succinate, maleate, gentisinate, fumarate, gluconate, glucaronate, saccharate, formate, benzoate, glutamate, methanesulfonate, ethanesulfonate, benzensulfonate, p-toluenesulfonate and pamoate (i.e., 1,1'-methylene-bis-(2-hydroxy-3-naphthoate)) salts. Certain compounds underlying the invention may form pharmaceutically acceptable salts with various amino acids. Suitable base salts include, but are not limited to, aluminum, calcium, lithium, magnesium, potassium, sodium, zinc, and diethanolamine salts. For a review on pharmaceutically acceptable salts see BERGE ET AL., 66 J. PHARM. SCI. 1-19 (1977).

The term "pharmaceutically acceptable topical formulation" as used herein means any formulation which is pharmaceutically acceptable for topical delivery of the compounds underlying the invention. A "topical formulation" will comprise at least a compound underlying the invention. The choice of topical formulation will depend on several factors, including the nature of the symptoms to be treated, the physiochemical characteristics of the particular compound underlying the invention and of other excipients present, their stability in the formulation, available manufacturing equipment, and cost constraints.

As used herein, a "therapeutically effective amount of a compound underlying the invention" means the minimum amount of the compound that is effective to treat the inflammatory dermatologic disorder.

As used herein, the term "subject" means any animal, preferably a mammal, to which will be or has been administered topical formulations underlying the invention. The term "mammal" as used herein, encompasses any mammal. Examples of mammals include, but are not limited to, cows, horses, sheep, pigs, cats, dogs, mice, rats, rabbits, guinea pigs, monkeys, humans etc., more preferably, a human. Preferably, a subject is in need of treatment of the inflammatory skin disorder and symptoms associated therewith.

The term "analog" refers to a chemical compound that is structurally similar to a parent compound and has chemical properties or pharmaceutical activity in common with the parent compound. Analogs include, but are not limited to, homologs, i.e., where the analog differs from the parent compound by one or more carbon atoms in series; positional isomers; compounds that differ by interchange of one or more atoms by a different atom, for example, replacement of a carbon atom with an oxygen, sulfur, or nitrogen atom; and compounds that differ in the identity of one or more functional groups, for example, the parent compound differs from its analog by the presence or absence of one or more suitable substituents. Suitable substituents include, but are not limited to, (C₁-C₈)alkyl; (C₁-C₈)alkenyl; (C₁-C₈)alkynyl: aryl; (C₂-C₅)heteroaryl; (C₁-C₆)heterocycloalkyl; (C₃-C₇)cycloalkyl; O-(C₁-C₈)alkyl; O-(C₁-C₈)alkenyl; O-(C₁-C₈)alkynyl; O-aryl; CN; OH; oxo; halo, C(O)OH; COhalo; O(CO)halo; CF₃, N₃; NO₂, NH₂; NH((C₁-C₈)alkyl); N((C₁-C₈)alkyl)₂; NH(aryl); N(aryl)₂ N((C₁-C₈)alkyl)(aryl); (CO)NH₂; (CO)NH((C₁-C₈)alkyl); (CO)N((C₁-C₈)alkyl)₂; (CO)NH(aryl); (CO)N(aryl)₂; O(CO)NH₂; NHOH; NOH((C₁-C₈)alkyl); NOH(aryl); O(CO)NH((C₁-C₈)alkyl); O(CO)N((C₁-C₈)alkyl)₂; O(CO)NH(aryl); O(CO)N(aryl)₂; CHO; CO((C₁-C₈)alkyl); CO(aryl); C(O)O((C₁-C₈)alkyl); C(O)O(aryl); O(CO)((C₁-C₈)alkyl); O(CO)(aryl); O(CO)O((C₁-C₈)alkyl); O(CO)O(aryl); S-(C₁-C₈)alkyl; S-(C₁-C₈)alkenyl; S-(C₁-C₈)alkynyl; S-aryl; S(O)-(C₁-C₈)alkyl; S(O)-(C₁-C₈)alkenyl; S(O)-(C₁-C₈)alkynyl; and S(O)-aryl; S(O)₂-(C₁-C₈)alkyl; S(O)₂-(C₁-C₈)alkenyl; S(O)₂-(C₁-C₈)alkynyl; and S(O)₂-aryl. One of skill in the art can readily choose a suitable substituent based upon the stability and pharmacological activity of the compound underlying the invention.

The term "derivative" refers to an analog, as defined above, that is synthesized in one or more chemical reactions from its parent compound.

A "hydrate" means a compound underlying the invention, or a pharmaceutically acceptable salt thereof that further includes a stoichiometric or non-stoichiometric amount of water bound to it by non-covalent intermolecular forces.

In one embodiment, "treatment" or "treating" refers to an amelioration, prophylaxis, or reversal of a disease or disorder, or at least one discernible symptom thereof. For example, treating an inflammatory skin disorder by lessening the redness of the skin. In another embodiment, "treatment" or "treating" refers to an amelioration, prophylaxis, or reversal of at least one measurable physical parameter related to the disease or disorder being treated, not necessarily discernible in or by the mammal. In yet another embodiment, "treatment" or "treating" refers to inhibiting or slowing the progression of a disease or disorder, either physically, e.g., stabilization of a discernible symptom, physiologically, e.g., stabilization of a physical parameter, or both.

As used herein, "carbomer" is the USP designation for various polymeric acids that are dispersible but insoluble in water. When the acid dispersion is neutralized with a base a clear, stable gel is formed. Carbomer 934P is physiologically inert and is not a primary irritant or sensitizer. Other carbomers include 910, 940, 941, and 1342.

## Claims

1. A composition comprising:
at least one of an α₂ adrenergic receptor agonist and a pharmaceutically acceptable salt thereof; and
a pharmaceutically acceptable carrier;
for use in the treatment of an inflammatory skin disorder and the symptoms associated therewith,
wherein said composition is to be topically applied directly to the affected area of skin of a patient in need of such treatment,
wherein the at least one of an α₂ adrenergic receptor agonist and a pharmaceutically acceptable salt thereof is selected from the group consisting of naphazoline and xylometazoline, and
wherein said inflammatory skin disorder is dermatitis, including contact dermatitis, atopic dermatitis, seborrheic dermatitis, nummular dermatitis, generalized exfoliative dermatitis, statis dermatitis, and lichen simplex chronicus.

2. Use of a composition comprising:
at least one of an α₂ adrenergic receptor agonist and a pharmaceutically acceptable salt thereof; and
a pharmaceutically acceptable carrier;
for the preparation of a medicament for treating an inflammatory skin disorder and the symptoms associated therewith,
wherein said composition is to be topically applied directly to the affected area of skin of a patient in need of such treatment,
wherein the at least one of an α₂ adrenergic receptor agonist and a pharmaceutically acceptable salt thereof is selected from the group consisting of naphazoline and xylometazoline, and
wherein said inflammatory skin disorder is dermatitis, including contact dermatitis, atopic dermatitis, seborrheic dermatitis, nummular dermatitis, generalized exfoliative dermatitis, statis dermatitis, and lichen simplex chronicus.

3. A use or the composition for use according to claim 1 or 2, wherein the pharmaceutically acceptable carrier is selected from the group consisting of pharmaceutically acceptable solvents; aqueous or non-aqueous solutions and suspensions; emulsions such as creams and lotions; gels; and ointments.

4. The composition for use according to claim 3, wherein the at least one of an α₂ adrenergic receptor agonist and a pharmaceutically acceptable salt thereof is present in an amount in the range of about 0.01 to 5 weight percent.

5. The composition for use according to claim 3, wherein the pH value of the composition is in the range of about 6 to 8.

6. The composition for use according to claim 1, further comprising a preservative.

7. The composition for use according to claim 1, further comprising a local anesthetic.

## Patentansprüche

1. Zusammensetzung umfassend:
mindestens einen eines α₂ adrenergen Rezeptoragonisten und eines pharmazeutisch verträglichen Salzes davon; und
einen pharmazeutisch verträglichen Träger;
zur Verwendung bei der Behandlung einer entzündlichen Hauterkrankung und der damit verbundenen Symptome,
wobei die Zusammensetzung topisch direkt auf den betroffenen Hautbereich eines Patienten, der einer solchen Behandlung bedarf, aufzutragen ist,
wobei der mindestens eine eines α₂ adrenergen Rezeptoragonisten und eines pharmazeutisch verträglichen Salzes davon ausgewählt ist aus der Gruppe bestehend aus Naphazolin und Xylometazolin, und
wobei die entzündliche Hauterkrankung Dermatitis, einschließlich Kontaktdermatitis, atopische Dermatitis, seborrhoische Dermatitis, nummuläre Dermatitis, generalisierte exfoliative Dermatitis, Stauungsekzem, und Lichen simplex chronicus, ist.

2. Verwendung einer Zusammensetzung umfassend:
mindestens einen eines α₂ adrenergen Rezeptoragonisten und eines pharmazeutisch verträglichen Salzes davon; und
einen pharmazeutisch verträglichen Träger;
zur Herstellung eines Medikaments zur Behandlung einer entzündlichen Hauterkrankung und der damit verbundenen Symptome,
wobei die Zusammensetzung topisch direkt auf den betroffenen Hautbereich eines Patienten, der einer solchen Behandlung bedarf, aufzutragen ist,
wobei der mindestens eine eines α₂ adrenergen Rezeptoragonisten und eines pharmazeutisch verträglichen Salzes davon ausgewählt ist aus der Gruppe bestehend aus Naphazolin und Xylometazolin, und
wobei die entzündliche Hauterkrankung Dermatitis, einschließlich Kontaktdermatitis, atopische Dermatitis, seborrhoische Dermatitis, nummuläre Dermatitis, generalisierte exfoliative Dermatitis, Stauungsekzem, und Lichen simplex chronicus, ist.

3. Verwendung oder die Zusammensetzung zur Verwendung nach Anspruch 1 oder 2, wobei der pharmazeutisch verträgliche Träger ausgewählt ist aus der Gruppe bestehend aus pharmazeutisch verträglichen Lösungsmitteln; wässrigen oder nichtwässrigen Lösungen und Suspensionen; Emulsionen wie Cremes und Lotionen; Gelen; und Salben.

4. Zusammensetzung zur Verwendung nach Anspruch 3, wobei der mindestens eine eines α₂ adrenergen Rezeptoragonisten und eines pharmazeutisch verträglichen Salzes davon in einer Menge im Bereich von etwa 0,01 bis 5 Gewichtsprozent vorhanden ist.

5. Zusammensetzung zur Verwendung nach Anspruch 3, wobei der pH-Wert der Zusammensetzung im Bereich von etwa 6 bis 8 liegt.

6. Zusammensetzung zur Verwendung nach Anspruch 1, ferner umfassend ein Konservierungsmittel.

7. Zusammensetzung zur Verwendung nach Anspruch 1, ferner umfassend ein Lokalanästhetikum.

## Revendications

1. Composition comprenant :
au moins un d'un agoniste des récepteurs α₂ adrénergiques et d'un sel pharmaceutiquement acceptable de celui-ci ; et
un support pharmaceutiquement acceptable ;
pour une utilisation dans le traitement d'un trouble cutané inflammatoire et des symptômes associés avec celui-ci,
dans laquelle ladite composition est destinée à être topiquement appliquée directement à la zone de peau affectée d'un patient nécessitant un tel traitement,
dans laquelle le au moins un d'un agoniste des récepteurs α₂ adrénergiques et d'un sel pharmaceutiquement acceptable de celui-ci est choisi dans le groupe constitué de naphazoline et xylométazoline, et
dans laquelle ledit trouble cutané inflammatoire est une dermatite, comprenant une dermatite de contact, une dermatite atopique, une dermatique séborrhéique, une dermatite nummulaire, une dermatite exfoliative généralisée, une dermatite statis, et une névrodermite circonscrite.

2. Utilisation d'une composition comprenant :
au moins un d'un agoniste des récepteurs α₂ adrénergiques et d'un sel pharmaceutiquement acceptable de celui-ci ; et
un support pharmaceutiquement acceptable ;
pour la préparation d'un médicament destiné au traitement d'un trouble cutané inflammatoire et aux symptômes associés avec celui-ci,
dans laquelle ladite composition est destinée à être topiquement appliquée directement à la zone de peau affectée d'un patient nécessitant un tel traitement,
dans laquelle le au moins un d'un agoniste des récepteurs α₂ adrénergiques et d'un sel pharmaceutiquement acceptable de celui-ci est choisi dans le groupe constitué de naphazoline et xylométazoline, et
dans laquelle ledit trouble cutané inflammatoire est une dermatite, comprenant une dermatite de contact, une dermatite atopique, une dermatite séborrhéique, une dermatite nummulaire, une dermatite exfoliative généralisée, une dermatite statis, et une névrodermite circonscrite.

3. Utilisation ou composition pour une utilisation selon la revendication 1 ou 2, dans laquelle le support pharmaceutiquement acceptable est choisi dans le groupe constitué de solvants pharmaceutiquement acceptables ; de solutions et de suspensions aqueuses ou non aqueuses, d'émulsions, telles que des crèmes et des lotions ; de gels ; et de baumes.

4. Composition pour une utilisation selon la revendication 3, dans laquelle le au moins un d'un agoniste des récepteurs α₂ adrénergiques et d'un sel pharmaceutiquement acceptable de celui-ci est présent dans une quantité dans l'intervalle d'environ 0,01 à 5 pourcents en masse.

5. Composition pour une utilisation selon la revendication 3, dans laquelle la valeur de pH de la composition se trouve dans l'intervalle d'environ 6 à 8.

6. Composition pour une utilisation selon la revendication 1, comprenant de plus un conservateur.

7. Composition pour une utilisation selon la revendication 1, comprenant de plus un anesthésique local.
